(19) Europäisches Patentamt
European Patent Office
Office européen des brevets

(11) **EP 0 566 925 B1**

(12) **EUROPÄISCHE PATENTSCHRIFT**

(45) Veröffentlichungstag und Bekanntmachung des Hinweises auf die Patenterteilung:
**03.07.1996  Patentblatt 1996/27**

(51) Int Cl.$^6$: **C07C 263/04**

(21) Anmeldenummer: **93105635.2**

(22) Anmeldetag: **06.04.1993**

(54) **Mehrstufiges Verfahren zur kontinuierlichen Herstellung von organischen Polyisocyanaten**

Multi-step method for the continuous preparation of organic polyisocyanates

Procédé de plusieurs étapes pour la préparation continue des polyisocyanates organiques

(84) Benannte Vertragsstaaten:
**AT BE CH DE DK ES FR GB IT LI NL PT SE**

(30) Priorität: **21.04.1992  DE 4213099**

(43) Veröffentlichungstag der Anmeldung:
**27.10.1993  Patentblatt 1993/43**

(73) Patentinhaber: **BASF Aktiengesellschaft**
**D-67063 Ludwigshafen (DE)**

(72) Erfinder:
- **Otterbach, Andreas, Dr.**
  **W-6710 Frankenthal (DE)**
- **Schwarz, Hans Volkmar, Dr.**
  **Baton Rouge 70 817 (US)**

- **Merger, Franz, Dr.**
  **W-6710 Frankenthal (DE)**
- **Schwarz, Wolfgang, Dr.**
  **W-6701 Otterstadt (DE)**
- **Brandt, Eckhardt, Dr.**
  **W-6707 Schifferstadt (DE)**
- **Magnussen, Peter, Dr.**
  **W-6702 Bad Duerkheim (DE)**
- **Mattner, Otto**
  **W-6720 Speyer (DE)**

(56) Entgegenhaltungen:
**EP-A- 0 054 817**     **EP-A- 0 126 299**
**EP-A- 0 355 443**     **DE-A- 3 314 790**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Gegenstand der Erfindung ist ein mehrstufiges Verfahren zur kontinuierlichen Herstellung von organischen, destillierbaren Polyisocyanaten, vorzugsweise von aliphatischen oder cycloaliphatischen Diisocyanaten, durch Umsetzung der entsprechenden organischen Polyamine mit Kohlensäurederivaten und Alkoholen in niedermolekulare monomere Polyurethane und deren thermische Spaltung, bei dem an bestimmten Reaktionsstufen die hergestellten Polyisocyanate und unverwertbaren Rückstände abgetrennt und wiederverwendbare Neben- und Zwischenprodukte in Vorstufen zurückgeführt werden.

Die technischen Verfahren zur Herstellung von organischen Polyisocyanaten, wie z.B. von aromatischen, aliphatischen oder cycloaliphatischen Polyisocyanaten, beruhen auf der Phosgenierung der entsprechenden organischen Polyamine zu Polycarbamidsäurechloriden und deren thermische Spaltung zu den Polyisocyanaten und Chlorwasserstoff. Abgesehen von den schwerwiegenden Umweltschutz-, Entsorgungs- und Sicherheitsproblemen, die der Einsatz von Phosgen mit sich bringt, sind diese Verfahren mit weiteren entscheidenden Nachteilen behaftet. So gelingt die Herstellung von aliphatischen oder cycloaliphatischen Polyisocyanaten aufgrund der stärkeren Basizität der Ausgangspolyamine nur mit recht mäßigen Raum-Zeit-Ausbeuten. Nachteilig ist ferner die Bildung von unerwünschten Nebenprodukten, die, bereits in Spuren vorliegend, zu starken Verfärbungen der Polyisocyanate führen können. Bei der Hexamethylen-diisocyanat-1,6(HDI)-Herstellung entstehen z.B. mehrere Nebenprodukte, von denen das wichtigste, 6-Chlorhexylisocyanat, zudem den Nachteil besitzt, daß es nur mit erheblichem destillativen Aufwand vom HDI abgetrennt werden kann.

Problematisch bei dieser Verfahrensweise sind insbesondere der hohe Umsatz von Chlor über Phosgen und Carbamidsäurechlorid in Chlorwasserstoff, die Toxizität des Phosgens sowie die Korrosivität des Reaktionsgemisches, die Labilität der in der Regel eingesetzten Lösungsmittel und die Bildung halogenhaltiger Rückstände.

Es hat daher nicht an Versuchen gefehlt organische Isocyanate, vorzugsweise aromatische und (cyclo)aliphatische Di- und/oder höherfunktionelle Polyisocyanate, nach einem phosgenfreien Verfahren herzustellen.

Zur Herstellung von aliphatischen und/oder cycloaliphatischen Di- und/oder Polyurethanen werden gemäß EP-A-0 018 588 (US-A-4 497 963) primäre aliphatische und/ oder cycloaliphatische Di- und/oder Polyamine mit O-Alkylcarbamidsäureestern in Gegenwart von Alkoholen im Verhältnis von $NH_2$-Gruppen der Amine zu Carbamidsäureestern zu Alkohol von 1:0,8 bis 10: 0,25 bis 50 bei Temperaturen von 160 bis 300°C in Gegenwart oder Abwesenheit von Katalysatoren umgesetzt und das entstehende Ammoniak gegebenenfalls abgetrennt. Die erhaltenen Di- und/oder Polyurethane können gegebenenfalls in die entsprechenden Di- und/ oder höherfunktionellen Polyisocyanate übergeführt werden. Detaillierte Reaktionsbedingungen zur Thermolyse werden in der Patentschrift nicht offenbart.

Nach Angaben der EP-A-28 338 (US-A-4 290 970) werden aromatische Di- und/oder Polyisocyanate hergestellt nach einem Zweistufenverfahren, wobei in der ersten Reaktionsstufe primäre aromatische Di- und/ oder Polyamine mit O-Alkylcarbamidsäureester in Abwesenheit oder Gegenwart von Katalysatoren und gegebenenfalls Harnstoff und Alkohol zu Aryl-di- und/oder -polyurethanen umgesetzt werden und das dabei gebildete Ammoniak gegebenenfalls abgetrennt wird und die erhaltenen Aryl-di- und/oder -polyurethane durch thermische Spaltung in der zweiten Reaktionsstufe in aromatische Di- und/oder Polyisocyanate übergeführt werden.

Weitere Veröffentlichungen beschäftigen sich mit der teilweisen Substitution von Harnstoff und/oder Diaminen durch carbonylgruppenhaltige Verbindungen, beispielsweise durch N-substituierte Carbamidsäureester und/oder Dialkylcarbonate bzw. mono- oder disubstituierte Harnstoffe oder Polyharnstoffe (EP-B-27 952 (US-A-4 388 238), EP-B-27 953 (US-A-4 430 505), EP-B-28 331 (US-A-4 480 110), EP-A-126 299 (US-A-4 596 678), EP-A-126 300 (US-A-4 596 679)).

Ein Verfahren zur Herstellung von aliphatischen O-Arylurethanen durch Umsetzung von (cyclo)aliphatischen Polyaminen mit Harnstoff und aromatischen Hydroxylverbindungen wird in der EP-A-0 320 235 beschrieben.

Obwohl die thermische Spaltung von (cyclo)aliphatischen und insbesondere aromatischen Mono- und Diurethanen in die entsprechenden Isocyanate und Alkohol seit langem bekannt ist und sowohl in der Gasphase bei hohen Temperaturen als auch in der flüssigen Phase bei vergleichsweise niedrigen Temperaturen ausgeführt werden kann, sind es besonders die unerwünschten Nebenreaktionen und vor allem die Tendenz der Reaktionsmischungen zur Ausbildung von Belegungen, Verharzungen und Verstopfungen in Reaktoren und Aufarbeitungseinrichtungen, die die Wirtschaftlichkeit der Prozesse nachhaltig beeinträchtigen.

Zahlreiche Patentpublikationen beschreiben daher beispielsweise chemische Methoden, wie z.B. den Einsatz von speziellen Katalysatoren (DE-C-1 022 222 (US-A-2 692 275) oder DE-B-19 44 719 (US-A-3 734 941)) oder Katalysatoren in Verbindung mit inerten Lösungsmitteln (US-A-3,919,279 oder DE-A-2 635 490 (US-A-4 081 472)) zur Verbesserung der Ausbeute bei der thermischen Urethanspaltung.

Die thermische Spaltung von Hexamethylendiethylurethan-1,6 unter Druck in Gegenwart von Dibenzyltoluol als Lösungsmittel und eines Katalysatorgemisches aus Toluolsulfonsäuremethylester und Diphenylzinndichlorid zur Herstellung von Hexamethylen-diisocyanat-1,6 wird z.B. in der DE-A-3 108 990 (US-A-4 388 246) beschrieben. Angaben über die Gewinnung der Ausgangskomponente, deren Isolierung sowie die Reinigung und gegebenenfalls Rückgewinnung des Lösungsmittels und der Katalysatormischung werden nicht gemacht. Berechnungen über die Wirtschaftlichkeit des Verfahrens sind daher nicht möglich.

Nach Angaben der EP-B-0 078 005 (US-A-4 482 499) können Urethane katalysatorfrei in einem Kohlenstoff enthaltenden Wirbelbett problemlos in das Isocyanat und Alkohol gespalten werden. Die Spaltung von Hexamethylen-dialkylurethanen zu Hexamethylen-diisocyanat kann gemäß DE-A-32 27 748 (US-A-4 613 466) in der Gasphase bei Temperaturen über 300°C gegebenenfalls in Gegenwart von Füllkörpern aus gasdurchlässigen Materialien, z.B. solchen aus Kohlenstoff, Stahl, Messing, Kupfer, Zink, Aluminium, Titan, Chrom, Kobalt oder Quarz oder nach Angaben der DE-A-32 48 018 (US-A-4 613 466) in Gegenwart von Halogenwasserstoffen und/oder Halogenwasserstoff-Donatoren durchgeführt werden.

Mit diesem Verfahren können jedoch keine Hexamethylen-diisocyanat-Ausbeuten von >90 % erzielt werden, da die Spaltprodukte teilweise rekombinieren. Bei der erforderlichen Reindestillation des Hexamethylen-diisocyanat-1,6 können sich die Ausbeuteverluste noch erhöhen.

Aus der EP-A-54 817 (US-A-4 386 033) ist ferner bekannt, daß sich Monocarbamate bei relativ niedrigen Temperaturen, vorzugsweise unter vermindertem Druck, gegebenenfalls in Gegenwart von Katalysatoren und/oder Stabilisatoren in guten Ausbeuten ohne den Einsatz von Lösungsmitteln spalten lassen, wobei die Spaltprodukte Monoisocyanat und Alkohol unter Sieden der Reaktionsmischung abdestillieren und durch fraktionierte Kondensation getrennt aufgefangen werden. In allgemeiner Form beschrieben werden auch Möglichkeiten einer Teilausschleusung der Reaktionsmischung zur Abtrennung der bei der thermischen Spaltung gebildeten Nebenprodukte. Eine mögliche technische Verwertung solcher Rückstände wird nicht erwähnt.

Nach Angaben der EP-A-0 061 013 (US-A-4 388 246) wird die thermische Spaltung von aliphatischen, cycloaliphatischen oder aromatischen Polycarbamaten bei Temperaturen von 150 bis 350°C und Drucken von 0,001 bis 20 bar in Gegenwart von inerten Lösungsmitteln, gegebenenfalls Katalysatoren sowie Chlorwasserstoff, organischen Säurechloriden, alkylierend wirkenden Substanzen oder Organozinn-IV-chloriden als Hilfsmitteln durchgeführt. Hierbei können die gebildeten Nebenprodukte z.B. mit der Reaktionslösung kontinuierlich aus dem Spaltreaktor entnommen und gleichzeitig eine entsprechende Menge an frischem oder zurückgewonnenem Lösungsmittel zudosiert werden. Nachteilig an diesem Verfahren ist beispielsweise, daß die Verwendung von unter Rückfluß siedendem Lösungsmittel zu einer Reduzierung der Raum-Zeit-Ausbeute an Polyisocyanaten führt und zusätzlich einen hohen Energieaufwand, z.B. auch zur Rückgewinnung der Lösungsmittel, erfordert. Außerdem können die eingesetzten, unter den Reaktionsbedingungen flüchtigen Hilfsmittel zur Verunreinigung der Spaltprodukte führen. Auffällig ist auch der auf das gebildete Polyisocyanat bezogene hohe Rückstandsanteil, der ebenso wie der niedrige Betriebsdruck eine wirtschaftliche und störungsfreie technische Fahrweise infrage stellt.

Ein Verfahren zur kontinuierlichen thermischen Spaltung von Carbamidsäureestern, wie z.B. des cycloaliphatischen Diurethans 5-(Ethoxycarbonylamino)-1-(ethoxycarbonylaminomethyl)-1,3,3-trimethylcyclohexan, wobei diese in Gegenwart eines hochsiedenden Lösungsmittels in flüssiger Form an der Innenwand eines Rohrreaktors entlang geführt werden, wird in der EP-B-92 738 (US-A-4 692 550) beschrieben. Nachteilig an diesem Verfahren sind die niedrigen Ausbeuten und die geringe Selektivität bei der Herstellung von (cyclo)aliphatischen Diisocyanaten. Ergebnisse einer kontinuierlichen Fahrweise unter Rückführung des rekombinierten oder teilweise gespaltenen Carbamidsäureesters werden ebensowenig offenbart wie Angaben über die Aufarbeitung des die Nebenprodukte und Katalysator enthaltenden Lösungsmittels.

Gegenstand der EP-A-0 355 443 ist ein Kreislaufverfahren zur Herstellung von (cyclo)aliphatischen Diisocyanaten durch Umwandlung der entsprechenden Diamine in Diurethane und deren thermische Spaltung, das Ausbeuteminderungen reduziert, indem der Austrag der Urethanspaltungsstufe nach Umsetzung mit Alkohol in die Urethanisierungsstufe zurückgeführt wird. Die Abtrennung von nicht rückführbaren Nebenprodukten erfolgt durch eine destillative Auftrennung des Urethanisierungsaustrages, in der der unverwertbare Rückstand als Sumpfprodukt anfällt und alle leichter siedenden Komponenten, u.a. auch das Diurethan, über Kopf abgezogen werden.

Nachteilig an dieser mit hohen Investitionskosten belasteten Verfahrensweise ist außerdem ihr hoher Energieaufwand, da das gesamte Diurethan in Gegenwart von Katalysatoren auf einem Temperaturniveau, das zudem im Bereich der Urethanspaltungstemperatur liegt, verdampft werden muß. Die sich bildenden Isocyanatgruppen des Wertproduktes reagieren mit den Urethangruppen des Rückstandes unter Bildung höhermolekularer, Ausbeute vermindernd wirkenden Nebenprodukten.

Die Aufgabe der vorliegenden Erfindung bestand darin, destillierbare organische Polyisocyanate, vorzugsweise aliphatische und cycloaliphatische Diisocyanate, mit hoher Selektivität in großen Raum-Zeit-Ausbeuten kostengünstig auf einfache Weise ohne die Verwendung von kostspieligen und/ oder sicherheitsgefährdenden Ausgangs- oder Hilfsstoffen herzustellen.

Diese Aufgabe konnte überraschenderweise gelöst werden mittels einer partiellen Ausschleusung von nichtverwertbaren Nebenprodukten vor der Polyurethanspaltung.

Gegenstand der Erfindung ist somit ein mehrstufiges Verfahren zur kontinuierlichen Herstellung von organischen Polyisocyanaten, vorzugsweise von aliphatischen oder cycloaliphatischen Diisocyanaten, durch Umsetzung der entsprechenden organischen Polyamine, vorzugsweise von aliphatischen oder cycloaliphatischen Diaminen, mit Kohlensäurederivaten und Alkoholen in Polyurethane, vorzugsweise Diurethane, und deren thermische Spaltung, das dadurch gekennzeichnet ist, daß man

a) organische Polyamine, vorzugsweise aliphatische oder cycloaliphatische Diamine, mit Harnstoff und Alkoholen in Abwesenheit oder vorzugsweise Gegenwart von Dialkylcarbonaten, Carbamidsäurealkylestern oder Mischungen aus Dialkylcarbonaten und Carbamidsäurealkylestern und in Abwesenheit oder vorzugsweise Gegenwart von Katalysatoren zu Polyurethanen, vorzugsweise Diurethanen, umsetzt und das entstehende Ammoniak gleichzeitig abtrennt,

b) aus der erhaltenen Reaktionsmischung den Alkohol, die Dialkylcarbonate und/oder Carbamidsäurealkylester abtrennt und vorzugsweise in die Reaktionsstufe a) zurückführt,

c) die Polyurethane, vorzugsweise aliphatische oder cycloaliphatische Diurethane, enthaltende Reaktionsmischung teilt, die eine Teilmenge destillativ trennt in ein Wertprodukt, das die Polyurethane, vorzugsweise Diurethane, und die leichter siedende Nebenprodukte enthält, und dieses Wertprodukt mit der anderen Teilmenge vereinigt, und einen unverwertbaren Rückstand, der aus dem Herstellungsverfahren abgetrennt wird,

d) die vereinigte, Polyurethane, vorzugsweise aliphatischen oder cycloaliphatischen Diurethane, enthaltende Reaktionsmischung teilweise, lösungsmittelfrei in flüssiger Phase in Gegenwart von Katalysatoren bei Temperaturen von 200 bis 300°C und unter einem Druck von 0,1 bis 200 mbar kontinuierlich thermisch spaltet und den ungespaltenen Anteil der Reaktionsmischung gemeinsam mit den gebildeten Nebenprodukten abtrennt und in die Reaktionsstufe a) zurückführt,

e) die Spaltprodukte durch Rekfifikation in ein rohes Polyisocyanat, vorzugsweise ein rohes aliphatisches oder cycloaliphatisches Diisocyanat, und Alkohol trennt und

f) das rohe Polyisocyanat, vorzugsweise das rohe aliphatische oder cycloaliphatische Diisocyanat, durch Destillation reinigt.

Nach einer bevorzugten Ausführungsform wird die bei der destillativen Reinigung des rohen Polyisocyanats (f) anfallende Kopffraktion in die Reaktionsstufe (a) zurückgeführt, die Seitenfraktion, die aus im wesentlichen reinem Polyisocyanat besteht, wird einem Behälter zur Lagerung zugeführt und die Sumpffraktion in die Reaktionsstufe (a) oder (d) oder (a) und (d) zurückgeführt.

Nach dem erfindungsgemäßen Verfahren können destillierbare organische Polyisocyanate, vorzugsweise aliphatische oder cycloaliphatische Diisocyanate, problemlos mit sehr guten Ausbeuten hergestellt werden. Vorteilhaft bei dem erfindungsgemäßen Mehrstufenverfahren ist insbesondere die einfache Auf- und Abtrennung bzw. Rückführung der intermediär gebildeten Dialkylcarbonate und/oder Carbamidsäurealkylester und des Alkohols sowie die Abtrennung der unverwertbaren hochsiedenden Nebenprodukte durch eine partielle Hochsiederausschleusung.

Rein formal betrachtet kann somit das erfindungsgemäße Verfahren schematisch durch folgende Gleichung bilanziert werden

$$R\text{-}(NH_2)_n + n\ H_2NCONH_2 + n\ ROH \rightarrow$$
$$R(NCO)_n + 2n\ NH_3 + n\ ROH$$

a) Zur Herstellung der monomeren Polyurethane, vorzugsweise (cyclo)aliphatischen Diurethane, in der Reaktionsstufe (a) werden die Polyamine, vorzugsweise Diamine, mit Harnstoff und einem Alkohol zweckmäßigerweise in einem Verhältnis von $NH_2$-Gruppe zu Harnstoff zu Alkohol von 1:0,9 bis 1,3:1 bis 5, vorzugsweise von 1:1,0 bis 1,2:1,5 bis 3 in Abwesenheit oder vorzugsweise Gegenwart von Dialkylcarbonaten oder vorzugsweise Carbamidsäureestern oder Mischungen aus Dialkylcarbonaten und Carbamidsäureestern sowie in Abwesenheit oder vorzugsweise Gegenwart von Katalysatoren bei Reaktionstemperaturen von 160 bis 300°C, vorzugsweise von 180 bis 250°C und insbesondere 185 bis 240°C und unter einem Druck, der in Abhängigkeit von dem verwendeten Alkohol zwischen 0,1 bis 60 bar, vorzugsweise 1 bis 40 bar liegt, zur Reaktion gebracht. Für diese Reaktionsbedingungen ergeben sich Reaktionszeiten von 0,5 bis 50, vorzugsweise 3 bis 15 Stunden.

Zur Herstellung der erfindungsgemäß als Zwischenprodukte verwendbaren monomeren Polyurethane eignen sich Amine der Formel $R(NH_2)_n$, in der R einen mehrwertigen, vorzugsweise zweiwertigen organischen Rest, wie z.B. einen gegebenenfalls substituierten, beispielsweise mit einer Alkylgruppe substituierten aromatischen oder vorzugsweise einen linearen oder verzweigtkettigen aliphatischen oder gegebenenfalls substituierten cycloaliphatischen Rest bedeutet. Als aromatische Polyamine beispielhaft genannt seien 2,4- und 2,6-Toluylen-diamin, 4,4'-, 2,4'- und 2,2'-Diamino-diphenylmethane und die entsprechenden Isomerengemische. Als aliphatische oder cycloaliphatische Polyamine kommen beispielsweise in Betracht: Butandiamin-1,4, 2-Ethylbutandiamin-1,4, Octandiamin-1,8, Decandiamin-1,10, Dodecandiamin-1,12, Cyclohexandiamin-1,4, 2-Methyl-, 4-Methyl-cyclohexandiamin-1,3, 1,3- und 1,4-Diaminomethylcyclohexan. Vorzugsweise Verwendung finden 2-Methylpentandiamin-1,5, 2,2,4-bzw. 2,4,4-Trimethylhexandiamin-1,6 und insbesondere Hexandiamin-1,6 und 3-Aminomethyl-3,5,5-trimethylcyclohexylamin.

Als Alkohole eignen sich prinzipiell alle aliphatischen Alkohole. Vorzugsweise wird man jedoch solche auswählen, deren Siedepunkte genügend weit vom Siedepunkt des durch die thermische Spaltung erhaltenen Polyisocyanates, vorzugsweise Diisocyanates, entfernt liegen, so daß eine möglichst quantitative Trennung der Spaltprodukte Polyisocyanat, vorzugsweise Diisocyanat und Alkohol möglich ist.

Aus diesen Gründen finden daher vorzugsweise Alkohole, wie z.B. Methanol, Ethanol, n-Propanol, n-Butanol, iso-Butanol, n-Pentanol, iso-Pentanol, n-Hexanol, Isohexa-nole, Cyclohexanol, 2-Ethylhexanol, Decanol oder Gemische der genannten Alkohole, insbesondere aber n-Butanol und/oder iso-Butanol Verwendung.

Wie bereits dargelegt wurde, wird die Umsetzung in der Reaktionsstufe (a) vorzugsweise in Gegenwart von Dialkylcarbonaten zweckmäßigerweise in einer Menge von 0,1 bis 30 Mol%, vorzugsweise 1 bis 10 Mol% oder vorzugsweise Carbamidsäurealkylestern zweckmäßigerweise in einer Menge von 1 bis 20 Mol%, vorzugsweise von 5 bis 15 Mol%, bezogen auf das Polyamin, vorzugsweise Diamin durchgeführt. Insbesondere verwendet werden jedoch Mischungen aus Dialkylcarbonaten und Carbamidsäurealkylestern in den genannten Mengenverhältnissen. Als Dialkylcarbonate und/oder Carbamidsäureester setzt man bevorzugt solche ein, deren Alkylreste dem Alkylrest des verwendeten Alkohols entsprechen.

Zur Erhöhung der Reaktionsgeschwindigkeit können die monomeren Polyurethane, vorzugsweise Diurethane, in Gegenwart von Katalysatoren hergestellt werden. Diese werden zweckmäßigerweise in Mengen von 0,01 bis 20 Gew.% vorzugsweise 0,05 bis 10 Gew.% und insbesondere 0,1 bis 5 Gew.%, bezogen auf das Gewicht des Polyamins, vorzugsweise Diamins, verwendet. Als Katalysatoren eignen sich anorganische oder organische Verbindungen, die ein oder mehrere Kationen, vorzugsweise ein Kation von Metallen der Gruppen IA, IB, IIA, IIB, IIIA, IIIB, IVA, IVB, VA, VB, VIB, VIIB, VIIIB des Periodensystems, definiert gemäß Handbook of Chemistry and Physics 14th Edition, publiziert von Chemical Rubber Publishing Co., 23 Superior Ave. N.E., Cleveland, Ohio, enthalten, beispielsweise Halogenide, wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocarbamate. Beispielhaft genannt seien die Kationen folgender Metalle: Lithium, Natrium, Kalium, Magnesium, Calcium, Aluminium, Gallium, Zinn, Blei, Bismut, Antimon, Kupfer, Silber, Gold, Zink, Quecksilber, Cer, Titan, Vanadium, Chrom, Molybdän, Mangan, Eisen und Cobalt. Die Katalysatoren können ohne erkennbare deutliche Nachteile auch in Form ihrer Hydrate oder Ammoniakate zum Einsatz kommen.

Als typische Katalysatoren seien beispielhaft folgende Verbindungen genannt: Lithiummethanolat, Lithiumethanolat, Lithiumpropanolat, Lithiumbutanolat, Natriummethanolat, Kalium-tert.-butanolat, Magnesiummethanolat, Calciummethanolat, Zinn-(II)-chlorid, Zinn-(IV)-chlorid, Bleiacetat, Bleiphosphat, Antimon-(III)-chlorid, Antimon-(V)-chlorid, Aluminiumacetylacetonat, Aluminium-isobutylat, Aluminiumtrichlorid, Bismut-(III)-chlorid, Kupfer-(II)-acetat, Kupfer-(II)-sulfat, Kupfer-(II)-nitrat, Bis-(triphenylphosphinoxido)-kupfer-(II)-chlorid, Kupfermolybdat, Silberacetat, Goldacetat, Zinkoxid, Zinkchlorid, Zinkacetat, Zinkacetonylacetat, Zinkoctoat, Zinkoxalat, Zinkhexylat, Zinkbenzoat, Zinkundecylenat, Cer-(IV)-oxid, Uranylacetat, Titantetrabutanolat, Titantetrachlorid, Titantetraphenolat, Titannaphtenat, Vanadium-(III)-chlorid, Vanadiumacetylacetonat, Chrom-(III)-chlorid, Molybdän-(VI)-oxid, Molybdänacetylacetonat, Wolfram-(VI)-oxid, Mangan-(II)-chlorid, Mangan-(II)-acetat, Mangan-(III)-acetat, Eisen-(II)-acetat, Eisen-(III)-acetat, Eisenphosphat, Eisenoxalat, Eisen-(III)-chlorid, Eisen-(III)-bromid, Cobaltacetat, Cobaltchlorid, Cobaltsulfat, Cobaltnaphthenat, Nickelchlorid, Nickelacetat und Nickelnaphthenat sowie deren Gemische.

Es hat sich als vorteilhaft erwiesen, das entstehende Ammoniak sofort aus der Reaktionsmischung, beispielsweise durch Destillation abzutrennen. Die hierfür verwendete Vorrichtung, beispielsweise eine Destillationskolonne, wird bei Temperaturen von 60 bis 150°C, vorzugsweise 65 bis 120°C, betrieben, so daß eine Belegung durch Ammoniumcarbaminat, welches in minimalen Mengen aus Ammoniak und Kohlendioxid durch Zersetzung von Harnstoff gebildet wird, vermieden werden kann.

b) Aus der durch vorteilhafterweise kontinuierliche Reaktion erhaltenen Reaktionsmischung (a) werden der Alkohol, die Dialkylcarbonate, sofern solche gebildet wurden oder in der Reaktionsmischung vorliegen, oder Carbamidsäurealkylester oder Mischungen aus mindestens zwei dieser Komponenten abgetrennt und vorzugsweise in die Reaktionsstufe (a) zurückgeführt. Zur Abtrennung der Komponenten wird die Reaktionsmischung vorteilhafterweise vom Druckniveau der Reaktionsstufe (a) auf einen Druck im Bereich von 1 bis 500 mbar, vorzugsweise von 10 bis 100 mbar entspannt. Man erhält hierbei gasförmige Brüden, die die überwiegende Alkoholmenge sowie 0 bis 30 Gew.%, vorzugsweise 1 bis 10 Gew.% Dialkylcarbonat und/oder 1 bis 50 Gew.%, vorzugsweise 1 bis 20 Gew.% Carbamidsäurealkylester enthalten, und einen flüssigen Austrag, der im wesentlichen aus dem monomeren Polyurethan, vorzugsweise Diurethan, besteht und gegebenenfalls Oligoharnstoff-polyurethanen und hochsiedende Oligomere enthält.

Die erhaltenen Brüden werden in nachfolgenden zweckmäßigerweise destillativen Reinigungsstufen, vorzugsweise durch Rektifikation, getrennt und die hierbei isolierten Wertprodukte Alkohol und Carbamidsäurealkylester, einzeln oder als Mischung, vorzugsweise in die Reaktionsstufe (a) zur Bildung der monomeren Polyurethane zurückgeführt.

c) Die in der Reaktionsstufe (b) nach Abtrennung der Brüden erhaltene flüssige, die monomeren Polyurethane, vorzugsweise Diurethane, und gegebenenfalls Oligoharnstoffpolyurethane und hochsiedende Oligomere enthaltende Reaktionsmischung (c) wird in zwei Teilströme geteilt, wobei das Gewichtsverhältnis der Teilmengen 5 bis 50:95 bis 50 Gew.-Teile, vorzugsweise 10 bis 30:90 bis 70 Gew.-Teile beträgt. Die gleich große oder vorzugsweise kleinere Teilmenge wird destillativ getrennt mittels einer üblichen Destillationsanlage, vorzugsweise eines Dünnschichtverdämpfers, bei einer Temperatur von 170 bis 240°C, vorzugsweise von 180 bis 230°C und unter einem Druck von 0,01 bis 5 mbar, vorzugsweise 0,1 bis 2 mbar, in ein Wertprodukt, das die Polyurethane, vorzugsweise Diurethane und die leichter siedende Nebenprodukte enthält, und nicht destillierbare Nebenprodukte, die aus dem Herstellungsverfahren abgetrennt und üblicherweise als nicht verwertbarer Rückstand verworfen werden. Das Wertprodukt wird mit der gleich großen oder vorzugsweise größeren anderen Teilmenge vereinigt und die vereinigte, Polyurethane, vorzugsweise Diurethane enthaltende Reaktionsmischung der thermischen Spaltung zugeführt.

Durch diese Verfahrensmaßnahme in der Reaktionsstufe (c) wird der Anteil an nicht destillierbaren Nebenprodukten in der Reaktionsmischung, die sich bei den nacheinander ablaufenden Teilreaktionen bilden und durch die Rückführung verwertbarer Einsatzstoff im Reaktionskreislauf ständig anreichern würden, auf einen Gehalt von 3 bis 30 Gew.%, vorzugsweise 5 bis 20 Gew.% begrenzt und dadurch eine mit hoher Selektivität störungsfrei ablaufende Reaktion gewährleistet.

d) Die in der Reaktionsstufe (c) erhaltene Polyurethane, vorzugsweise Diurethane, enthaltende Reaktionsmischung wird in einer geeigneten Vorrichtung, teilweise, lösungsmittelfrei in flüssiger Phase in Gegenwart von Katalysatoren bei Temperaturen von 200 bis 300°C, vorzugsweise 220 bis 280°C und unter vermindertem Druck von 0,1 bis 200 mbar, vorzugsweise 5 bis 80 mbar kontinuierlich thermisch gespalten. Der Umsatz von Polyurethan zu Polyisocyanat, vorzugsweise von Diurethan zu Diisocyanat, in der Vorrichtung zur thermischen Spaltung kann in Abhängigkeit vom verwendeten Polyurethan weitgehend frei gewählt werden und liegt zweckmäßigerweise in einem Bereich von 10 bis 95 Gew.%, vorzugsweise 40 bis 85 Gew.% der zugeführten Polyurethanmenge. Der ungespaltene Anteil der Reaktionsmischung, der nicht umgesetzte Polyurethane, Oligoharnstoff-polyurethane, hochsiedende Oligomere und andere wieder verwertbare und unverwertbare Nebenprodukte enthält, wird abgetrennt, kontinuierlich aus der Spaltvorrichtung ausgeschleust und direkt oder gegebenenfalls nach Umsetzung mit Alkohol in die Reaktionsstufe (a) zurückgeführt.

Als Katalysatoren zur chemischen Spaltung der Polyurethane finden z.B. die vorgenannten, die Urethanbildung katalysierende anorganischen und organischen Verbindungen Verwendung.

Besonders bewährt und daher vorzugsweise verwendet werden Dibutylzinndilaurat, Eisen-(III)-acetylacetonat, Kobalt-(II)-acetylacetonat, Zinkacetylacetonat und Zinn-(II)-dioctoat.

Als Spaltvorrichtungen eignen sich beispielsweise zylinderförmige Spaltreaktoren, wie z.B. Röhrenöfen oder vorzugsweise Verdampfer, beispielsweise Dünnschicht- oder Bulkverdampfer, wie z.B. Robertverdampfer, Herbertverdampfer, caddle-typ-Verdampfer und vorzugsweise Heizkerzenverdampfer.

e) Die bei der thermischen Spaltung gebildeten Spaltprodukte, die sich vor allem aus Alkohol, Polyisocyanat, vorzugsweise Diisocyanat, und partiell gespaltenen Polyurethanen zusammensetzen, werden danach vorteilhafterweise mit Hilfe einer oder mehrerer Destillationskolonnen, vorzugsweise durch Rektifikation bei Temperaturen von 100 bis 220°C, vorzugsweise 120 bis 170°C und einem Druck von 1 bis 200 mbar, vorzugsweise 5 bis 50 mbar, in Alkohol und eine rohe Polyisocyanatmischung mit einem Polyisocyanatgehalt von 85 bis 99 Gew.%, vorzugsweise von 95 bis 99 Gew.% getrennt. Die bei der destillativen Trennung anfallenden höhersiedenden Nebenprodukte und insbesondere die ungespaltenen und partiell gespaltenen Polyurethane werden vorzugsweise in die Spaltvorrichtung zurückgeführt.

f) Die vorzugsweise durch Rektifikation erhaltene rohe Polyisocyanatmischung wird durch Destillation bei einer Temperatur von 100 bis 180°C und unter einem Druck von 1 bis 50 mbar gereinigt, wobei die einzelnen Fraktionen zurückgeführt oder als Reinprodukt isoliert werden. Wie bereits ausgeführt wurde, wird bei der bevorzugt angewandten Reindestillation die Kopffraktion, die vorzugsweise aus Polyisocyanat, insbesondere Diisocyanat besteht, gegebenenfalls nach Umsetzung der freien Isocyanatgruppen mit Alkohol in die Reaktionsstufe (a), die Polyurethanbildung, zurückgeführt, die Seitenfraktion, die aus reinem Polyisocyanat, insbesondere Diisocyanat, vorzugsweise mit einer Reinheit von mindestens 98 Gew.%, insbesondere über 99 Gew.% besteht, wird abgeleitet und der Lagerung zugeführt und die Sumpffraktion, die als wesentliche Komponenten die partiell gespaltenen Polyurethane und Polyisocyanate enthält, wird vorzugsweise in die Spaltvorrichtung zur thermischen Spaltung zurückgeführt. Nach anderen Verfahrensvarianten kann die Sumpffraktion jedoch auch in die Destillationskolonne (e) zur Trennung von rohem Polyisocyanat und Alkohol oder in der Reaktionsstufe (a), die Polyurethanbildung zurückgeführt werden. Möglich ist auch eine Teilung der Sumpffraktion in 2 oder 3 Produktströme, wobei diese vorzugsweise in der Polyurethanbildung (a) und die Spaltvorrichtung (d) sowie gegebenenfalls in die Destillationskolonne (e) zurückgeführt werden.

Mit dem erfindungsgemäßen mehrstufigen Verfahren zur kontinuierlichen Herstellung von organischen Polyisocyanaten unter Rückführung und Ausschleusung der Nebenprodukte können destillierbare Polyisocyanate, vorzugsweise Diisocyanat mit hoher Selektivität in sehr guten Ausbeuten hergestellt werden. Das erfindungsgemäße Verfahren eignet sich insbesondere zur Herstellung von aliphatischen Diisocyanaten, wie 2-Methylpentan-diisocyanat-1,5, isomeren aliphatischen Diisocyanaten mit 6 Kohlenstoffatomen im Alkylenrest und deren Gemische und vorzugsweise Hexamethylen-diisocyanat-1,6 und cycloaliphatischen Diisocyanaten, insbesondere 3-Isocyanatomethyl-3,5,5-trimethylcyclohexylisocyanat nach einer wirtschaftlichen Methode.

Die hergestellten Polyisocyanate eignen sich vorzüglich zur Herstellung von Urethan-, Isocyanurat-, Amid- und/oder Harnstoffgruppen enthaltenden Kunststoffen nach dem Polyisocyanat-Polyadditionsverfahren. Sie finden ferner Verwendung zur Herstellung von mit Urethan-, Biuret- und/oder Isocyanuratgruppen modifizierten Polyisocyanatmischungen. Derartige Polyisocyanatmischungen aus aliphatischen oder cycloaliphatischen Diisocyanaten werden insbesondere zur Herstellung von lichtbeständigen Polyurethanlacken und -überzügen verwendet.

Beispiel 1

In den ersten Reaktionsbehälter einer dreistufigen Rührkesselkaskade, ausgerüstet mit aufgesetzten beheizten Kolonnen und Kopfkondensatoren sowie einer Druckhaltung, in der sich eine Mischung aus Hexamethylendibutylurethan-1,6 und n-Butanol neben Hexamethylen-oligo-harnstoff-polybutylurethanen, Dibutylcarbonat und Carbamidsäurebutylester befand, fügte man bei 220-230°C und einem Druck von 12 bar während einer Stunde 0,879 kg Harnstoff, 0,805 kg Hexamethylendiamin-1,6 und 0,089 kg n-Butanol sowie 3,333 kg einer Produktmischung, die enthielt Spaltbutanol, einen Teil der Reaktionsmischung aus der Urethanspaltung mit den gebildeten Nebenprodukten, die hauptsächlich bestanden aus höhermolekularen Isocyanurat-, Allophanat-, Harnstoff- und Polyurethangruppenhaltigen Verbindungen, und die Kopffraktion der Hexamethylendiisocyanat-Reindestillation. Das aus der unter Rückfluß siedenden Reaktionsmischung entweichende, gebildete Ammoniak wurde über die Kolonnen abgetrennt und in den nachgeschalteten Kondensatoren durch fraktionierte Kondensation nahezu quantitativ vom Butanol befreit.

Der Reaktionsaustrag aus dem 3. Kessel der Rührkesselkaskade wurde kontinuierlich in einen bei 50 mbar betriebenen Behälter entspannt. Die gasförmigen Brüden gelangten direkt in eine ebenfalls bei 50 mbar betriebene Rektifikationskolonne an deren Kopf ca. 2,8 kg/h n-Butanol, im Seitenabzug 0,06 kg/h eines dibutylcarbonatreichen Azeotropes sowie im Abtriebsteil 0,234 kg/h Carbamidsäurebutylester anfielen. Das n-Butanol und der Carbamidsäurebutylester wurden in die Rührkesselkaskade zurückgeführt.

Der flüssige Austrag des Entspannungsbehälters wurde ungefähr im Gewichtsverhältnis 3 : 1 aufgeteilt und der kleinere Anteil einem Dünnschichtverdampfer zugeführt. Betrieben bei 220°C und 1 mbar fielen im Sumpf 0,071 kg/h unverdampfbarer Rückstand an (Hochsiederausschleusung). Das am Kopf kondensierte Hexamethylendibutylurethan-1,6 wurde mit dem größeren Anteil des flüssigen Austrages des Entspannungsbehälters vereinigt, die über den Rückstand zwangsweise ausgeschleuste Katalysatormenge an Dibutylzinndilaurat ergänzt und über eine Dosiervorrichtung im schmelzflüssigen Zustand einem dampfbeheizten Verdampferreaktor mit 2,5 l Reaktionsvolumen für die homogen katalysierte thermische Spaltung zugeführt. Die Spaltung mit einem Umsatz von ca. 55 % bezüglich der eingesetzten 3,81 kg/h Hexamethylendibutylurethan-1,6 erfolgte bei 30 mbar unter intensivem Sieden des Reaktionsgemisches. Die gasförmigen Brüden gelangten zur Auftrennung in eine Rektifizierkolonne, an deren Kopf 1,1 kg/h flüssiges Spaltbutanol abgenommen wurden. Im Seitenabzug fiel ein ca. 95 gew. %iges Rohdiisocyanat an. Ungespaltenes Diurethan und 6-Isocyanatohexylbutylurethan wurden in den Verdampferreaktor zurückgeführt.

Das so gewonnene rohe Hexamethylen-diisocyanat-1,6 wurde einer Reindestillation unterzogen, wobei im Seitenabzug einer bei 30 mbar betriebenen Kolonne 1,115 kg/h Hexamethylendiisocyanat-1,6 mit einer Reinheit von > 99 % anfiel. Der Sumpf der Reindestillation, der sich vorwiegend aus 6-Isocyanatohexylbutylurethan und dessen höhermolekularen Oligomeren zusammensetzte, wurde direkt in den Verdampferreaktor bzw. in die sich anschließende Rektifizierkolonne zurückgeführt.

Das Kopfprodukt der Reindestillation vereinigt mit dem Spaltbutanol und dem die hochsiedenden Nebenprodukte enthaltenden Ablauf aus dem Verdampferreaktor zur Urethanspaltung gelangte direkt zurück in die Reaktionsstufe a), der dreistufigen Rührkesselkaskade. Die Gesamtselektivität für die Umwandlung von eingesetztem Hexamethylen-diamin-1,6 zu Hexamethylendiisocyanat-1,6 betrug 97 %.

Beispiel 2

Es wurde analog den Angaben von Beispiel 1 verfahren, wobei sich jedoch in der Rührkesselkaskade anstelle von Hexamethylendiamin-Derivaten entsprechende 2-Methylpentamethylen-1,5-diamin-Derivate, Dibutylcarbonat und Carbamidsäurebutylester befanden. Zu dieser Mischung fügte man bei 220 bis 230°C und einem Druck von 12 bar während einer Stunde 0,886 kg Harnstoff, 0,810 kg 2-Methylpentamethylen-1,5-diamin und 0,097 kg n-Butanol sowie 3,543 kg einer Produktmischung aus Spaltbutanol, einem Teil der Reaktionsmischung aus der Urethanspaltung und der Kopffraktion der Diisocyanat-Reindestillation.

Der Reaktionsaustrag aus dem 3. Kessel der Rührkesselkaskade wurde bei 50 mbar entspannt und die Brüden gasförmig in eine Rektifikationskolonne geführt, an deren Kopf ca. 2,8 kg/h n-Butanol, im Seitenabzug 0,06 kg/h eines dibutylcarbonatreichen Azeotropes sowie im Abtriebsteil 0,245 kg Carbamidsäurebutylester anfielen. Das n-Butanol und der Carbamidsäurebutylester wurden in die Rührkesselkaskade zurückgeführt.

Im Sumpf des Dünnschichtverdampfers zur partiellen Hochsiederausschleusung fielen 0,087 kg/h unverdampfbarer Rückstand an.

Die Urethanspaltung mit einem Umsatz von ca. 55 % bezüglich der eingesetzten 3,80 kg/h 2-Methylpentamethylen-1,5-diurethan erfolgte bei 30 mbar. Am Kopf der nachgeschalteten Rektifikationskolonne fielen 1,1 kg/h flüssiges Spaltbutanol und im Seitenabzug ein ca. 95 gew.%iges Rohdiisocyanat an. Nach der Reindestillation ergaben sich 1,110 kg/h 2-Methylpentamethylen-1,5-diisocyanat. Daraus berechnete sich eine Gesamtselektivität für die Umwandlung von eingesetztem 2-Methylpentamethylen-1,5-diamin zu 2-Methylpentamethylen-1,5-diisocyanat von 96 %.

Beispiel 3

Es wurde analog den Angaben von Beispiel 1 verfahren, wobei sich jedoch in der Rührkesselkaskade anstelle von Hexamethylendiamin-Derivaten entsprechende 3-Aminomethyl-3,5,5-trimethylcyclohexylamin-Derivate, Dibutylcarbonat und Carbamidsäurebutylester befanden. Zu dieser Mischung fügte man bei 220 bis 230°C und einem Druck von 12 bar während einer Stunde 0,625 kg Harnstoff, 0,839 kg 3-Aminomethyl-3,5,5-trimethylcyclohexylamin und 0,061 kg n-Butanol sowie 2,297 kg einer Produktmischung aus Spaltbutanol, einem Teil der Reaktionsmischung aus der Urethanspaltung und der Kopffraktion der Diisocyanat-Reindestillation.

Der Reaktionsaustrag aus dem 3. Kessel der Rührkesselkaskade wurde bei 50 mbar entspannt und die Brüden gasförmig in eine Rektifikationskolonne geführt, an deren Kopf ca. 2,1 kg/h n-Butanol, im Seitenabzug 0,05 kg/h eines dibutylcarbonatreichen Azeotropes sowie im Abtriebsteil 0,175 kg Carbamidsäurebutylester anfielen. Das n-Butanol und der Carbamidsäurebutylester wurden in die Rührkesselkaskade zurückgeführt.

Im Sumpf des Dünnschichtverdampfers zur partiellen Hochsiederausschleusung fielen 0,044 kg/h unverdampfbarer Rückstand an.

Die Urethanspaltung mit einem Umsatz von ca. 60 % bezüglich der eingesetzten 2,94 kg/h 3-Urethanomethyl-3,5,5-trimethylcyclohexylurethan erfolgte bei 20 mbar. Am Kopf der nachgeschalteten Rektifikationskolonne fielen 0,81 kg/h flüssiges Spaltbutanol und im Seitenabzug ein ca. 95 gew.%iges Rohdiisocyanat an. Nach der Reindestillation ergaben sich 1,060 kg/h 3-Isocyanatomethyl-3,5,5-trimethylcyclohexylisocyanat. Daraus berechnete sich eine Gesamtselektivität für die Umwandlung von eingesetztem 3-Aminomethyl-3,5,5-trimethylcyclohexylamin zu 3-Isocyanatomethyl-3,5,5-trimethylcyclohexylisocyanat von 98 %.

Beispiel 4

Es wurde analog den Angaben von Beispiel 1 verfahren, wobei sich jedoch in der Rührkesselkaskade anstelle von Hexamethylendiamin-Derivaten entsprechende 2,2,4 (2,4,4)-Trimethylhexamethylendiamin-1,6-Derivate, Dibutylcarbonat und Carbamidsäurebutylester befanden. Zu dieser Mischung fügte man bei 220 bis 230°C und einem Druck von 12 bar während einer Stunde 0,746 kg Harnstoff, 0,923 kg 2,2,4 (2,4,4)-Trimethylhexamethylendiamin-1,6 und 0,088 kg n-Butanol sowie 3,379 kg einer Produktmischung aus Spaltbutanol, einem Teil der Reaktionsmischung aus der Urethanspaltung und der Kopffraktion der Diisocyanat-Reindestillation.

Der Reaktionsaustrag aus dem 3. Kessel der Rührkesselkaskade wurde bei 50 mbar entspannt und die Brüden gasförmig in eine Rektifikationskolonne geführt, an deren Kopf ca. 2,9 kg/h n-Butanol, im Seitenabzug 0,06 kg/h eines dibutylcarbonatreichen Azeotropes sowie im Abtriebsteil 0,239 kg Carbamidsäurebutylester anfielen. Das n-Butanol und der Carbamidsäurebutylester wurden in die Rührkesselkaskade zurückgeführt.

Im Sumpf des Dünnschichtverdampfers zur partiellen Hochsiederausschleusung fielen 0,074 kg/h unverdampfbarer Rückstand an.

Die Urethanspaltung mit einem Umsatz von ca. 50 % bezüglich der eingesetzten 3,99 kg/h 2,2,4 (2,4,4)-Trimethylhexamethylendiamin-1,6 erfolgte bei 30 mbar. Am Kopf der nachgeschalteten Rektifikationskolonne fielen 0,95 kg/h flüssiges Spaltbutanol und im Seitenabzug ein ca. 95 gew.%iges Rohdiisocyanat an. Nach der Reindestillation ergaben sich 1,170 kg/h 2,2,4 (2,4,4)-Trimethylhexamethylen-diisocyanat-1,6. Daraus berechnete sich eine Gesamtselektivität für die Umwandlung von eingesetztem 2,2,4 (2,4,4)-Trimethylhexamethylendiamin-1,6 zu 2,2,4 (2,4,4)-Trimethylhexamethylen-diisocyanat-1,6 von 97 %.

Beispiel 5

Es wurde analog den Angaben von Beispiel 1 verfahren, wobei jedoch zur Durchführung der Urethanisierungsreaktion ein Reaktionssystem bestehend aus einem Reaktionskessel mit aufgesetzter beheizter Kolonne und Kopfkondensator sowie einer nachgeschalteten Reaktionskolonne mit 10 Böden zum Einsatz kam. Am Fuß der Reaktionskolonne wurden ca. 2,8 kg/h n-Butanoldampf eingespeist und gasförmig im Gegenstrom zum flüssigen Produktstrom geführt. Die n-Butanolbrüden vom Kopf der Reaktionskolonne gelangten direkt in den Brüdenraum des Reaktionskessels.

Die Einsatzstoffe und die Rückführströme aus den anderen Reaktionsstufen wurden in den Reaktionskessel dosiert.

Der flüssige Reaktionsaustrag aus der Reaktionskolonne wurde kontinuierlich in einen bei 50 mbar betriebenen Behälter entspannt.

Beispiel 6

Es wurde analog den Angaben von Beispiel 1 verfahren, wobei jedoch die aus der Urethansynthese erhaltene Reaktionsmischung in eine bei 500 mbar betriebene Kolonne geführt wurde. Am Kopf dieser n-Butanol-Kolonne wurden ca. 1,7 kg/h n-Butanol abgenommen, nicht kondensiert, sondern nach Überhitzung in den Sumpf einer nachgeschalteten, bei 100 mbar betriebenen Stripp-Kolonne geführt. Der Sumpf der n-Butanol-Kolonne wurde am Kopf der Stripp-Kolonne aufgegeben und durch den im Gegenstrom aufsteigenden n-Butanol-Dampf von den Restmengen n-Butanol, vor allem aber Dibutylcarbonat und Carbamidsäurebutylester befreit.

Der flüssige Austrag der Strippkolonne wurde ungefähr im Gewichtsverhältnis 3:1 aufgeteilt und sowohl dem Dünnschichtverdampfer zur partiellen Hochsiederausschleusung als auch der Urethanspaltung zugeführt.

Die gasförmigen Brüden der Strippkolonne gelangten in eine bei 50 mbar betriebene Rektifikationskolonne zur Auftrennung in die in Beispiel 1 genannten Austragsströme n-Butanol, Dibutylcarbonat und Carbamidsäurebutylester.

Beispiel 7

Es wurde analog den Angaben von Beispiel 1 verfahren, wobei jedoch die gasförmigen Brüden aus der Urethanspaltung in eine erste Rektifikationskolonne geführt wurden, an deren Kopf gasförmiges n-Butanol und Diisocyanat anfielen. Dieser gasförmige Strom gelangte in die Reindestillationskolonne, bestehend aus einer Hauptkolonne, an deren Kopf 1,1 kg/h flüssiges Spaltbutanol anfielen und einer Seitenkolonne, die im Seitenabzug 1,115 kg/h Hexamethylen-diisocyanat-1,6 lieferte.

Das n-Butanol-Kopfprodukt der Hauptkolonne sowie das Kopfprodukt der Seitenkolonne vereinigt mit dem die hochsiedenden Nebenprodukte enthaltenden Ablauf aus dem Verdampferreaktor zur Urethanspaltung gelangte direkt zurück in die Reaktionsstufe a), der dreistufigen Rührkesselkaskade.

Der Sumpf der Reindestillationskolonne diente als Rücklauf für die der Urethanspaltung nachgeschaltete Rektifikationskolonne.

**Patentansprüche**

1. Mehrstufiges Verfahren zur kontinuierlichen Herstellung von organischen Polyisocyanaten durch Umsetzung der entsprechenden organischen Polyamine mit Kohlensäurederivaten und Alkoholen in Polyurethane und deren thermische Spaltung, dadurch gekennzeichnet, daß man

a) organische Polyamine mit Harnstoff und Alkoholen in Abwesenheit oder Gegenwart von Dialkylcarbonaten und/oder Carbamidsäurealkylestern und in Gegenwart oder Abwesenheit von Katalysatoren zu Polyurethanen umsetzt und das entstehende Ammoniak gleichzeitig abtrennt,

b) aus der erhaltenen Reaktionsmischung den Alkohol, die Dialkylcarbonate und/oder Carbamidsäurealkylester abtrennt und vorzugsweise in die Reaktionsstufe a) zurückführt,

c) die Polyurethane enthaltende Reaktionsmischung teilt, die eine Teilmenge destillativ trennt in ein Wertprodukt, das die Polyurethane und die leichter siedende Nebenprodukte enthält, und dieses Wertprodukt mit der anderen Teilmenge vereinigt, und einen unverwertbaren Rückstand, der aus dem Herstellungsverfahren abgetrennt wird,

d) die vereinigte, Polyurethane enthaltende Reaktionsmischung teilweise, lösungsmittelfrei in flüssiger Phase in Gegenwart von Katalysatoren bei Temperaturen von 200 bis 300°C und unter einem Druck von 0,1 bis 200 mbar kontinuierlich thermisch spaltet und den ungespaltenen Anteil der Reaktionsmischung gemeinsam mit den gebildeten Nebenprodukten abtrennt und in die Reaktionsstufe a) zurückführt,

e) die Spaltprodukte durch Rektifikation in eine rohe Polyisocyanatmischung und Alkohol trennt und

f) das rohe Polyisocyanat durch Destillation reinigt.

2. Mehrstufiges Verfahren nach Anspruch 1, dadurch gekennzeichnet, daß das rohe Polyisocyanat (f) durch Destillation gereinigt wird, wobei

die Kopffrakfion in die Reaktionsstufe (a) zurückgeführt wird,

die Seitenfraktion aus im wesentlichen reinem Polyisocyanat besteht und

die Sumpffraktion in die Reaktionsstufe (a) oder (d) oder (a) und (d) zurückgeführt wird.

3. Mehrstufiges Verfahren zur kontinuierlichen Herstellung von aliphatischen oder cycloaliphatischen Diisocyanaten durch Umsetzung der entsprechenden Diamine mit Kohlensäurederivaten und Alkoholen in Diurethane und deren thermische Spaltung, dadurch gekennzeichnet, daß man

a) aliphatische oder cycloaliphatische Diamine mit Harnstoff und Alkoholen in Abwesenheit oder Gegenwart von Dialkylcarbonaten und/oder Carbamidsäurealkylestern und in Gegenwart oder Abwesenheit von Katalysatoren zu Diurethanen umsetzt und das entstehende Ammoniak gleichzeitig abtrennt,

b) aus der erhaltenen Reaktionsmischung den Alkohol, die Dialkylcarbonate und/oder Carbamidsäurealkylester abtrennt und vorzugsweise in die Reaktionsstufe a) zurückführt,

c) die Diurethane enthaltende Reaktionsmischung teilt, die eine Teilmenge destillativ trennt in ein Wertprodukt, das die Diurethane und die leichter siedende Nebenprodukte enthält, und dieses Wertprodukt mit der anderen Teilmenge vereinigt, und einen unverwertbaren Rückstand, der aus dem Herstellungsverfahren abgetrennt wird,

d) die vereinigte, Diurethane enthaltende Reaktionsmischung teilweise, lösungsmittelfrei in flüssiger Phase in Gegenwart von Katalysatoren bei Temperaturen von 200 bis 300°C und unter einem Druck von 0,1 bis 200 mbar kontinuierlich thermisch spaltet und den ungespaltenen Anteil der Reaktionsmischung gemeinsam mit den gebildeten Nebenprodukten abtrennt und in die Reaktionsstufe a) zurückführt,

e) die Spaltprodukte durch Rekfifikation in eine rohe Diisocyanatmischung und Alkohol trennt und

f) das rohe Diisocyanat durch Destillation reinigt.

4. Mehrstufiges Verfahren nach Anspruch 3, dadurch gekennzeichnet, daß das rohe Diisocyanat (e) durch Destillation gereinigt wird, wobei

die Kopffraktion in die Reaktionsstufe (a) zurückgeführt wird,

die Seitenfraktion aus im wesentlichen reinem Diisocyanat besteht und

die Sumpffraktion in die Reaktionsstufe (a) oder (d) oder (a) und (d) zurückgeführt wird.

5. Mehrstufiges Verfahren nach Anspruch 3, dadurch gekennzeichnet, daß man die Diurethane enthaltende Reaktionsmischung (c) im Gewichtsverhältnis 5 bis 50:95 bis 50 teilt und die erstgenannte kleinere Teilmenge destillativ trennt.

6. Mehrstufiges Verfahren nach Anspruch 3, dadurch gekennzeichnet, daß man die erhaltene Reaktionsmischung (b) zur Abtrennung des Alkohols, der Dialkylcarbonate und/oder Carbamidsäurealkylester von 1 bis 40 bar auf 1 bis 500 mbar entspannt.

7. Mehrstufiges Verfahren nach Anspruch 3, dadurch gekennzeichnet, daß man als aliphatische Diamine Hexamethylendiamin-1,6 und/oder isomere aliphatische Diamine mit 6 Kohlenstoffatomen im Alkylenrest verwendet.

8. Mehrstufiges Verfahren nach Anspruch 3, dadurch gekennzeichnet, daß man als cycloaliphatische Diamine 3-Aminomethyl-3,5,5-trimethyl-cyclohexylamin verwendet.

**Claims**

1. A multistep process for the continuous preparation of organic polyisocyanates by reacting the corresponding organic polyamines with carbonic acid derivatives and alcohols to give polyurethanes, and thermolysis thereof, which comprises

a) reacting organic polyamines with urea and alcohols in the presence or absence of dialkyl carbonates and/or alkyl carbamates and in the presence or absence of catalysts to give polyurethanes, and simultaneously removing the resultant ammonia,

b) removing the alcohol, the dialkyl carbonates and/or alkyl carbamates from the resultant reaction mixture and preferably recycling them into reaction step a),

c) dividing the polyurethane-containing reaction mixture, separating one part, by distillation, into a useful product, which contains the polyurethanes and the relatively low-boiling by-products and is then combined with the other part of the reaction mixture, and a worthless residue, which is removed from the preparation process,

d) continuously thermolyzing some of the combined, polyurethane-containing reaction mixture in the liquid phase in the absence of solvents in the presence of catalysts at from 200 to 300°C and at from 0.1 to 200 mbar, and removing the unthermolyzed component of the reaction mixture together with the resultant by-products and recycling these into reaction step a),

e) separating the thermolysis products into a crude polyisocyanate mixture and alcohol by rectification, and

f) purifying the crude polyisocyanate by distillation.

2. A multistep process as claimed in claim 1, wherein the crude polyisocyanate (f) is purified by distillation, where

the top fraction is recycled into reaction step (a),

the side fraction comprises essentially pure polyisocyanate, and

the bottom fraction is recycled into reaction step (a) or (d) or (a) and (d).

3. A multistep process for the continuous preparation of aliphatic or cycloaliphatic diisocyanates by reacting the corresponding diamines with carbonic acid derivatives and alcohols to give diurethanes, and thermolysis thereof, which comprises

a) reacting aliphatic or cycloaliphatic diamines with urea and alcohols in the presence or absence of dialkyl carbonates and/or alkyl carbamates and in the presence or absence of catalysts to give diurethanes, and simultaneously removing the resultant ammonia,

b) removing the alcohol, the dialkyl carbonates and/or alkyl carbamates from the resultant reaction mixture and preferably recycling them into reaction step a),

c) dividing the diurethane-containing reaction mixture, separating one part, by distillation, into a useful product, which contains the diurethanes and the relatively low-boiling by-products and is then combined with the other part of the reaction mixture, and a worthless residue, which is removed from the preparation process,

d) continuously thermolyzing some of the combined, diurethane-containing reaction mixture in the liquid phase in the absence of solvents in the presence of catalysts at from 200 to 300°C and at from 0.1 to 200 mbar, and removing the unthermolyzed component of the reaction mixture together with the resultant by-products and recycling these into reaction step a),

e) separating the thermolysis products into a crude diisocyanate mixture and alcohol by rectification, and

f) purifying the crude diisocyanate by distillation.

4. A multistep process as claimed in claim 3, wherein the crude diisocyanate (e) is purified by distillation, where

the top fraction is recycled into reaction step (a),

the side fraction comprises essentially pure diisocyanate, and

the bottom fraction is recycled into reaction step (a) or (d) or (a) and (d).

5. A multistep process as claimed in claim 3, wherein the diurethane-containing reaction mixture (c) is divided in a weight ratio of from 5 to 50:95 to 50, and the first-mentioned, relatively small part of the reaction mixture is separated by distillation.

6. A multistep process as claimed in claim 3, wherein the resultant reaction mixture (b) is decompressed from between 1 and 40 bar to between 1 and 500 mbar in order to remove the alcohol, the dialkyl carbonates and/or alkyl carbamates.

7. A multistep process as claimed in claim 3, wherein the aliphatic diamines used are hexamethylene-1,6-diamine and/or isomeric aliphatic diamines having 6 carbon atoms in the alkylene radical.

8. A multistep process as claimed in claim 3, wherein the cycloaliphatic diamines used are 3-aminomethyl-3,5,5-trimethylcyclohexylamine.

**Revendications**

1. Procédé en plusieurs étapes pour la préparation en continu de polyisocyanates organiques par conversion des polyamines organiques correspondantes avec des dérivés d'acides carboxyliques et des alcools en polyuréthannes et leur coupure thermique, caractérisé en ce que

a) on fait réagir des polyamines organiques avec de l'urée et des alcools en l'absence ou en présence de dialkylcarbonates et/ou d'esters alkyliques d'acide carbamique et en présence ou en l'absence de catalyseurs pour former des polyuréthannes et on sépare simultanément l'ammoniac formé,

b) on sépare du mélange réactionnel obtenu l'alcool, les dialkylcarbonates et/ou les esters alkyliques d'acide carbamique et on les réintroduit de préférence dans l'étape réactionnelle a),

c) on divise le mélange réactionnel contenant les polyuréthannes, on sépare par distillation l'une des fractions en un produit de valeur, contenant les polyuréthannes et les sous-produits de plus faible point d'ébullition, et on réunit ce produit de valeur avec l'autre fraction, et un résidu non valorisable, qui est éliminé du procédé de préparation,

d) on coupe thermiquement, en continu, le mélange réactionnel réuni, contenant des polyuréthannes, en l'absence de solvant, en phase liquide, en présence de catalyseurs, à des températures de 200 à 300°C et sous une pression de 0,1 à 200 mbar et on sépare la portion non coupée du mélange réactionnel conjointement avec les sous-produits formés et on les recycle dans l'étape réactionnelle a),

e) on sépare les produits de coupure par rectification en un mélange brut de polyisocyanate et de l'alcool et

f) purifie le polyisocyanate brut par distillation.

2. Procédé en plusieurs étapes selon la revendication 1, caractérisé en ce que le polyisocyanate brut (f) est purifié par distillation, tandis que

la fraction de tête est recyclée dans l'étape réactionnelle (a),
la fraction latérale consiste en du polyisocyanate pratiquement pur et
la fraction pâteuse est recyclée dans l'étape réactionnelle (a) ou (d) ou (a) et (d).

3. Procédé en plusieurs étapes pour la préparation en continu de diisocyanates aliphatiques ou cycloaliphatiques par conversion des diamines correspondantes avec des dérivés d'acide carboxylique et des alcools en des diuréthannes et la coupure thermique de ceux-ci, caractérisé en ce que

a) on fait réagir des diamines aliphatiques ou cycloaliphatiques avec de l'urée et des alcools en l'absence ou en présence de dialkylcarbonates et/ou d'esters alkyliques d'acide carbamique et en présence ou en l'absence de catalyseurs pour former des diuréthannes et on sépare simultanément l'ammoniac formé,

b) on sépare du mélange réactionnel formé l'alcool, les dialkylcarbonates et/ou les esters dialkyliques d'acide carbamique et on les recycle éventuellement dans l'étape réactionnelle a),

c) on divise le mélange réactionnel contenant les diuréthannes, on sépare par distillation l'une des fractions en un produit de valeur, contenant les diuréthannes et les sous-produits de plus faible point d'ébullition, et on réunit ce produit de valeur avec l'autre fraction, et un résidu non valorisable, qui est éliminé du procédé de préparation,

d) on coupe thermiquement, en continu, le mélange réactionnel réuni, contenant des diuréthannes, en l'absence de solvant, en phase liquide, en présence de catalyseurs, à des températures de 200 à 300°C et sous une pression de 0,1 à 200 mbar et on sépare la portion non coupée du mélange réactionnel conjointement avec les sous-produits formés et on les recycle dans l'étape réactionnelle a),

e) on sépare les produits de coupure par rectification en un mélange brut de diisocyanate et de l'alcool et

f) on purifie le diisocyanate brut par distillation.

4. Procédé en plusieurs étapes selon la revendication 3, caractérisé en ce que le diisocyanate brut (e) est purifié par distillation, tandis que

la fraction de tête est recyclée dans l'étape réactionnelle (a),
la fraction latérale consiste en du diisocyanate pratiqueent pur et
la fraction pâteuse est recyclée dans l'étape réactionnelle (a) ou (d) ou (a) et (d).

5. Procédé en plusieurs étapes selon la revendication 3, caractérisé en ce qu'on partage le mélange réactionnel (c) contenant les diuréthannes dans la proportion en poids de 5 à 50:95 à 50 et on sépare par distillation les fractions plus petites mentionnées en premier.

6. Procédé en plusieurs étapes selon la revendication 3, caractérisé en ce qu'on détend le mélange réactionnel (b) obtenu pour séparer l'alcool, les dialkylcarbonates et/ou les esters alkyliques d'acide carbamique de 1 à 40 bar à 1 à 500 mbar.

7. Procédé en plusieurs étapes selon la revendication 3, caractérisé en ce qu'on utilise, comme diamines aliphatiques, de l'hexaméthylènediamine-1,6 et/ou des diamines aliphatiques isomères ayant 6 atomes de carbone dans le reste alkylène.

8. Procédé en plusieurs étapes selon la revendication 3, caractérisé en ce qu'on utilise, comme diamines cycloaliphatiques, de la 3-aminométhyl-3,5,5-triméthylcyclohexylamine.